# EUROPÄISCHE PATENTSCHRIFT

(11) **EP 3 684 299 B1**
(45) Veröffentlichungstag und Bekanntmachung des Hinweises auf die Patenterteilung: **29.06.2022**
(21) Anmeldenummer: 18769380.9
(22) Anmeldetag: 20.09.2018
(51) Int. Cl.: A61F 5/01, A61F 2/58, A61H 1/02, A61F 2/30, A61F 2/50

(54) **MODULGLIED ZUR VERWENDUNG IN EINER HANDORTHESE**
MODULE MEMBER FOR USE IN A HAND ORTHOSIS
ÉLÉMENT MODULAIRE DESTINÉ À L'UTILISATION DANS UNE ORTHÈSE À MAIN

(30) Priorität: 20.09.2017 EP 17192053
(43) Veröffentlichungstag der Anmeldung: 29.07.2020
(73) Patentinhaber: HKK Bionics GmbH, 89075 Ulm (DE)
(72) Erfinder: HEPP, Dominik, 89231 Neu-Ulm (DE); KNOBLOCH, Tobias, 89077 Ulm (DE)
(74) Vertreter: Hentrich Patent- & Rechtsanwälte PartG mbB
(86) Internationale Anmeldenummer: PCT/EP2018/075448
(87) Internationale Veröffentlichungsnummer: WO 2019/057811

(56) Entgegenhaltungen:
- WO-A1-03/017879
- WO-A2-2006/063347
- US-A- 2 545 452
- US-A- 2 582 234
- US-A1- 2008 188 952

## Beschreibung

Die Erfindung ist durch Anspruch 1 definiert und betrifft ein Modulglied für eine Handorthese. Die Handorthese dient zum Beugen und/oder Strecken mindestens eines Fingers einer Patientenhand, mit einem an einer Schiene befestigten Krafteinleitungsmittel, das mit mindestens einem dem Finger der Patientenhand zugeordneten Fingersegment gekoppelt ist, wobei das mindestens eine Fingersegment eine Mehrzahl von gelenkig miteinander verbundenen Modulgliedern umfasst.

Derartige Orthesen werden üblicherweise bei Schlaganfallpatienten oder anderen Patienten mit eingeschränkter motorischer Fähigkeit dazu verwendet, deren Finger aktiv zu bewegen. Hierdurch wird diesen die Möglichkeit gegeben, ihre Finger trotz der fehlenden motorischen Fähigkeit zu verstellen und damit Gegenstände wieder sicher greifen zu können. Derartige Handorthesen sind daher ein wichtiges Instrument, um den Patienten eine gewisse Selbstständigkeit zu ermöglichen und ihnen damit ein Stück Lebensqualität zurück zu geben.

Die WO 03 / 017 879 A1 beschreibt eine vollständige mechanische Hand, also eine Prothese, die aus einer Mehrzahl von Modulen gebildet ist. Auch in der US 2 582 234 A wird eine künstliche Hand und damit eine Prothese beschrieben, die aus einzelnen Fingersegmenten besteht. Die US 2 545 452 beschreiben einen künstlichen Finger, dessen einzelnen Segmente durch eine Stiftverbindung gekoppelt sind.

Handorthesen der eingangs genannten Art sind aus dem Stand der Technik beispielsweise aus der WO 2016/088071 bereits bekannt. Die

Fingersegmente der darin gezeigten Orthese sind aus einzelnen Modulgliedern aufgebaut, die durch eine Kette miteinander gekoppelt sind. Durch die Verbindung der Modulglieder über die Kette ist es hierbei möglich, den axialen Abstand einzelner Modulglieder relativ zueinander zu verstellen. Hierbei hat es sich jedoch als äußerst nachteilig erwiesen, dass die Kette zwischen den Modulgliedern sehr aufwendig in der Herstellung und Montage ist und zudem auch in relativ hohen und klobigen Fingersegmenten resultiert. Dies birgt aber die Gefahr, dass der Patient beispielsweise beim Überziehen einer Jacke mit der Handorthese in deren Ärmel hängen bleibt, so dass die Orthese letztlich hinderlich ist und der Patient gezwungen ist, die Orthese häufiger abzulegen.

Die aus der US 5,178,137 bekannte Orthese weist Fingersegmente auf, die aus äußeren und inneren Modulgliedern gebildet sind, welche fest miteinander verbunden sind und schwenkbar gegeneinander verstellt werden können. Durch einen Spindeltrieb lassen sich dabei die Fingersegmente beugen und strecken. Hierbei hat es sich jedoch als nachteilig erwiesen, dass sich bei der Verwendung der aus der US 5,178,137 bekannten Fingersegmente diese durch den Spindeltrieb nur sehr langsam verstellen lassen. Zudem ist die Anpassung der Fingersegmente an unterschiedlich lange Finger nur schwer möglich und die Mechanik der darin gezeigten Fingersegmente neigt auch zum Verklemmen.

Es ist die Aufgabe der vorliegenden Erfindung, ein verbessertes Modulglied für die Verwendung in einer derartigen Handorthese anzugeben.

Das erfindungsgemäße Modulglied weist an einer ersten Stirnseite mindestens eine Bajonettkulisse und an einer zweiten Stirnseite mindestens einen zu der Bajonettkulisse korrespondierenden Bajonetthaken auf zur Bildung einer ein Bajonettgelenk bereitstellenden Bajonettverbindung zwischen zwei benachbarten Modulgliedern.

Hierdurch ist es möglich, die einzelnen Modulglieder einfach gelenkig miteinander zu verbinden, während gleichzeitig sichergestellt ist, dass sich die durch die Bajonettverbindung miteinander verbundenen Modulglieder nicht voneinander lösen können, wenn diese in der Handorthese eingebaut sind, da für die Lösung der Bajonettverbindung eine Relativdrehung zwischen den durch die Bajonettverbindung gesicherten Modulgliedern notwendig ist, was im eingebauten Zustand nur schwer möglich ist. Hierbei hat es sich als besonders günstig erwiesen, wenn der Drehwinkel zur Bildung und Lösung der Bajonettverbindung zwischen 40° und 90°, bevorzugt zwischen 50° und 70° und besonders bevorzugt bei 65° liegt. Hierdurch wird sichergestellt, dass sich die Bajonettverbindung, die benachbarte Modulglieder miteinander verbindet, nicht unbeabsichtigt lösen kann.

Um hierbei die Gelenkigkeit zwischen den durch die Bajonettverbindung verbundenen Modulgliedern zu fördern hat es sich zudem als günstig erwiesen, wenn die Bajonettkulisse und der Bajonetthaken jeweils mehrfach vorgesehen und unter einem Winkel zueinander angeordnet sind, der zwischen 40° und 170°, bevorzugt zwischen 60° und 160° und besonders bevorzugt bei 120° liegt. Indem die Bajonettkulissen und -haken auf einem gedachten Kreis, der in einer senkrecht zur Fingerachse liegenden Ebene liegt, nicht gegenüberliegend, was einem Winkel von 180° entspräche, sondern unter einem davon abweichenden Winkel angeordnet sind, wird durch die Bajonettverbindung letztlich ein Bajonettgelenk realisiert, das eine einfache Verkippung benachbarter Modulglieder relativ zueinander ermöglicht. Hierbei hat es sich besonders bewährt, wenn die Bajonettkulisse und der Bajonetthaken zweifach vorgesehen sind.

Es kann hierbei jedoch auch vorgesehen sein, dass die Bajonettkulisse und der Bajonetthaken jeweils dreifach vorgesehen sind.

Bewährt hat es sich zudem auch, wenn die Bajonettverbindung benachbarter Modulglieder derartig ausgestaltet ist, dass eine axiale Verstellung in Längsrichtung des Fingersegments der durch die Bajonettverbindung verbundenen Modulglieder relativ zueinander ermöglicht ist. Hierdurch wird auf einfache Art und Weise sichergestellt, dass die bei der Beugung der Finger der Patientenhand auftretende Längenänderungen auf der Oberseite der Finger durch die Modulglieder kompensiert werden können, da diese durch die Bajonettverbindung nicht nur schwenkbar, sondern auch in axialer Richtung beweglich ausgestaltet sind. Dies wird dabei vorteilhafterweise durch die Gestaltung der Bajonettkulisse erreicht, die zu diesem Zweck eine Hakenaufnahme aufweist, in der sich der korrespondierende Bajonetthaken in seiner finalen Position aufgenommen ist und dort axial verstellen lässt. Die Bajonetthaken werden also zunächst in die Bajonettkulisse eingeführt und folgen dieser. Dabei werden die beiden benachbarten Modulglieder um den Drehwinkel verstellt und aufeinander zu bewegt. Wenn die finale Position erreicht ist, befinden sich die Bajonetthaken in den Hakenaufnahmen, so dass sich die benachbarten Modulglieder in axialer Richtung gegeneinander verstellen lassen. Die Bajonetthaken und die Bajonettkulissen bilden zudem einen die Bewegung begrenzenden Anschlag und korrespondierenden Gegenanschlag, die eine Verkippung einzelner Modulglieder relativ zueinander begrenzen, wodurch ein Herausspringen der Modulglieder verhindert wird.

Als besonders bevorzugt hat es sich zudem auch gezeigt, wenn an der ersten Stirnseite der Modulglieder jeweils eine Aufnahme ausgebildet ist zur Aufnahme eines jeweils an der zweiten Stirnseite benachbarter Modulglieder ausgebildeten Vorsprungs, derartig, dass eine Überdachung zwischen benachbarten Modulgliedern gebildet wird. Durch die Überdachung wird insbesondere erreicht, dass ein Auswölben der die Kraft von dem Krafteinleitungsmittel auf die Fingersegmente übertragenden Kopplungselemente effektiv unterbunden wird. Ansonsten bestünde bei einem zu hohen Druck die Gefahr einer plastischen Verformung der Kopplungselemente. Zudem lässt sich durch die Überdachung sicherstellen, dass auch bei einem gebeugten Finger keine Spalte entstehen, die anderenfalls mit einer Einklemmungsgefahr verbunden wären. Letztlich ist durch die Überdachung auch ein Schutz der Kopplungselemente gegeben.

Als vorteilhaft hat es sich auch erwiesen, wenn dem distalen Ende der Schiene ein Handschuhabschnitt zugeordnet ist, und wenn eine Palmarstütze vorgesehen ist, die den Handschuhabschnitt auf dessen der Handfläche zugewandten Seite umgreift und die an dem distalen Ende der Schiene zumindest teilweise lösbar befestigt ist. Hierdurch wird ein besonders einfaches Anlegen der Handorthese möglich, da der Patient lediglich den Handschuhabschnitt an der betroffenen Hand anziehen und die Schiene am Unterarm befestigen muss. Um hierbei der Hand im Bereich des Handgelenks zusätzliche Stabilität zu verleihen, muss der Patient sodann lediglich die Palmarstütze über die Handinnenfläche des angezogenen Handschuhabschnitts herumlegen und an der Schiene befestigen. Dies kann dabei beispielsweise durch eine Rastverbindung erfolgen. Zur Fixierung der Schiene am Unterarm des Patienten hat sich hierbei insbesondere ein Klettband oder ein elastisches Band bewährt, das an der Schiene beispielsweise über eine Rastverbindung gesichert wird. Dabei kann es vorgesehen sein, dass die Palmarstütze an einer Seite der Schiene gelenkig befestigt ist - beispielsweise durch ein Scharnier - und auf der anderen Seite über die Rastverbindung an der Schiene gesichert wird.

Für den Herstellungsprozess hat es sich zudem als günstig erwiesen, wenn die Palmarstütze aus einem Niedertemperaturthermoplast-Material und wenn der Handschuhabschnitt aus Silikon gefertigt sind. Während bei der Herstellung der Schiene ein 3D-Scanverfahren verwendet werden kann, mit dem die Physiologie des Unterarms und zumindest eines Teils der Handoberseite sehr genau erfasst und damit die Schiene mittels eines 3D Druckers in einem generativen Herstellungsprozess kundenspezifisch hergestellt werden kann, ist die Erfassung der Geometrie der Handinnenfläche, die für eine generative Erstellung der Palmarstütze mittels 3D-Drucker notwendig wäre, nur schwer durchzuführen, so dass hier die patientenspezifische Anpassung bzw. Anfertigung manuell erfolgen muss. Der Handschuhabschnitt wird dabei basierend auf einem Gipsmodell der Patientenhand aus Silikon gefertigt. Die Verwendung von Silikon für den Handschuhabschnitt bringt dabei zudem den Vorteil eines angenehmen Tragekomforts mit sich. Der Handschuhabschnitt ist dabei durch die Fingersegmente mit der Schiene verbunden kann aber auch zusätzlich noch durch eine Schraubverbindung daran befestigt werden.

Als günstig hat es sich weiterhin auch gezeigt, wenn das Fingersegment ein distales Endstück und ein proximales Endstück umfasst, wobei das proximale Endstück an der Schiene und das distale Endstück und die Modulglieder an dem Handschuhabschnitt befestigt sind. Zwischen den proximalen und dem distalen Endstück sind dabei die Modulglieder angeordnet, die ebenfalls über eine Bajonettverbindung mit dem proximalen bzw. distalen Endstück verbunden sind. Hierzu ist an einer Stirnseite des einen Endstücks eine Bajonettkulisse und an einer Stirnseite des anderen Endstücks ein Bajonetthaken ausgebildet. Durch die Befestigung des proximalen Endstücks an der Schiene einerseits und die Befestigung des distalen Endstücks an dem Handschuhabschnitt andererseits lässt sich zudem eine Ausrichtung der Fingersegmente erreichen. Das proximale Endstück kann dabei auch teilweise an dem Handschuhabschnitt befestigt sein.

Zur Verankerung der Modulglieder in dem Handschuhabschnitt hat es sich zudem auch bewährt, wenn den Modulgliedern jeweils mindestens ein Flügel zugeordnet ist. Insbesondere wenn der Handschuhabschnitt aus Silikon gefertigt ist, kann durch die Verwendung der Flügel eine verbesserte Einbettung der Modulglieder in das verwendete Silikon erfolgen, wobei es sich in diesem Zusammenhang noch zusätzlich bewährt hat, wenn dem Flügel jeweils eine Öffnung und/oder ein Widerhaken zugeordnet ist. Durch die Öffnung kann insbesondere zusätzliches Silikonmaterial eingepresst werden, wodurch insbesondere in Kombination mit den Widerhaken ein verbesserter Halt der Modulglieder in dem Handschuhabschnitt realisiert ist. Die Widerhaken können jedoch auch seitlich an den Modulgliedern benachbart zu den Flügeln ausgebildet sein. Auch können die Flügel selbst hakenförmig gebildet sein. Durch die Einbettung der Modulglieder in das Material des Handschuhabschnitts wird zudem die translatorische Bewegung benachbarter Modulglieder gehemmt und gleichzeitig wirkt diese als zusätzlicher Drehpunkt.

Um ein Aufbiegen der Außenwand zu verhindern, das gegebenenfalls dazu führen würde, dass der Bajonetthaken aus der Führung springt und sich die Modulglieder weiter als gewünscht abwinkeln, hat es sich als vorteilhaft gezeigt, wenn die lateralen Bereiche der Modulglieder gegenüber der Oberseite und/oder Unterseite verstärkt sind.

Um die Lebensdauer der Handorthese zu erhöhen, hat es sich zudem als vorteilhaft erwiesen, wenn in der Schiene Kanäle ausgebildet sind zur Aufnahme von Kopplungselementen, die ausgelegt sind, Zug und/oder Druckkräfte von dem Krafteinleitungsmittel auf die Fingersegmente zu übertragen. Hierdurch ist es möglich, die Kopplungselemente vollständig in der Schiene aufzunehmen, so dass die Gefahr reduziert wird, dass diese beschädigt werden. Außerdem lässt sich damit eine besonders kompakte Handorthese erreichen. Bezüglich der Kopplungselemente ist es insbesondere vorgesehen, dass diese draht- oder stabförmig gebildet sind und aus einem Metall oder aus einer Metalllegierung, wie beispielsweise Nitinol, gefertigt sind. Die Kopplungselemente können dabei runde und unrunde, insbesondere auch flache Querschnitte aufweisen.

Für die Montage hat es sich zudem auch bewährt, wenn in der Schiene Aussparungen eingearbeitet sind zur Aufnahme der Krafteinleitungsmittel und/oder der proximalen Enden der Fingersegmente. Insbesondere können dabei auch in der Schiene Gewindebuchsen eingearbeitet sein, in die die Krafteinleitungsmittel und/oder die proximalen Endstücke der Fingersegmente festgeschraubt werden können. Durch die Aussparung ist zudem die Montage der Krafteinleitungsmittel bzw. der Fingersegmente deutlich erleichtert und die Handorthese kann deutlich flacher gebildet werden.

Die das Modulglied betreffende Aufgabe wird gemäß der Erfindung durch ein Modulglied mit den Merkmalen des Anspruchs 1 gelöst, nämlich mit mindestens einer an einer ersten Stirnseite ausgebildeten Bajonettkulisse und mit mindestens einem zu der Bajonettkulisse korrespondierenden Bajonetthaken, der an einer zweiten Stirnseite ausgebildet ist, zur Bildung einer Bajonettverbindung zwischen benachbarten Modulgliedern. Bevorzugte Ausgestaltungen des Modulglieds sind in den abhängigen Ansprüchen angegeben.

Hierdurch wird ein Modulglied bereitgestellt, das einfach mit benachbarten Modulgliedern verbunden werden kann, während gleichzeitig durch die Gestaltung der Bajonettverbindung und der Bajonettkulisse sowie durch die Anordnung der Bajonetthaken sowohl ein Verkippen benachbarter Modulglieder aber auch deren translatorische Verstellung relativ zueinander möglich ist.

Ein Herstellungsverfahren umfasst die Schritte:
- Erstellen eines Handschuhabschnitts,
- Adaption der Länge der Fingersegmente an die Finger der Patientenhand durch Auswahl der Anzahl der verwendeten Modulglieder,
- Einbetten der Modulglieder in das Obermaterial des Handschuhabschnitts,
- Erfassen der Physiologie des Unterarms und/oder der Hand des Patienten mittels eines 3D Scanners,
- Erstellen einer Schiene auf Grundlage der ermittelten Physiologie, vorzugsweise durch ein generatives Herstellungsverfahren,
- Befestigen der proximalen Endstücke der Fingersegmente an dem distalen Ende der Schiene,
- Anpassen der Palmarstütze an die individuelle Handinnenfläche des Patienten,
- Befestigen der Krafteinleitungsmittel auf der Schiene und
- Koppeln der Krafteinleitungsmittel mit den Fingersegmenten über die Kopplungsmittel.

Hierdurch wird ein Verfahren bereitgestellt, das eine individuell an die jeweilige Patientenhand angepasste Handorthese bereitstellt, die äußerst angenehm zu tragen ist und gleichzeitig eine hohe Funktionalität bietet.

Im Folgenden wird die Erfindung an einem in der Zeichnung dargestellten Ausführungsbeispiel näher erläutert; es zeigen:
- Fig. 1: eine perspektivische Ansicht einer Handorthese,
- Fig. 2: eine perspektivische Ansicht auf eine erste Stirnseite eines Modulgliedes der Handorthese,
- Fig. 3: eine perspektivische Ansicht auf eine zweite Stirnseite des Modulgliedes,
- Fig. 4: eine Draufsicht auf die erste Stirnseite des Modulgliedes,
- Fig. 5: eine geschnittene Ansicht durch das Modulglied,
- Fig. 6: eine perspektivische Ansicht auf ein Fingersegment,
- Fig. 7: einen Längsschnitt durch das Fingersegment,
- Fig. 8: eine Schnittansicht durch ein gestrecktes Fingersegment,
- Fig. 9: eine Schnittansicht durch ein gebeugtes Fingersegment,
- Fig. 10: einen Querschnitt durch ein an einem Handschuhabschnitt befestigtes Fingersegment,
- Fig. 11: eine Draufsicht auf eine Schiene einer Handorthese, und
- Fig. 12: eine Seitenansicht auf die in der Figur 11 gezeigte Schiene.

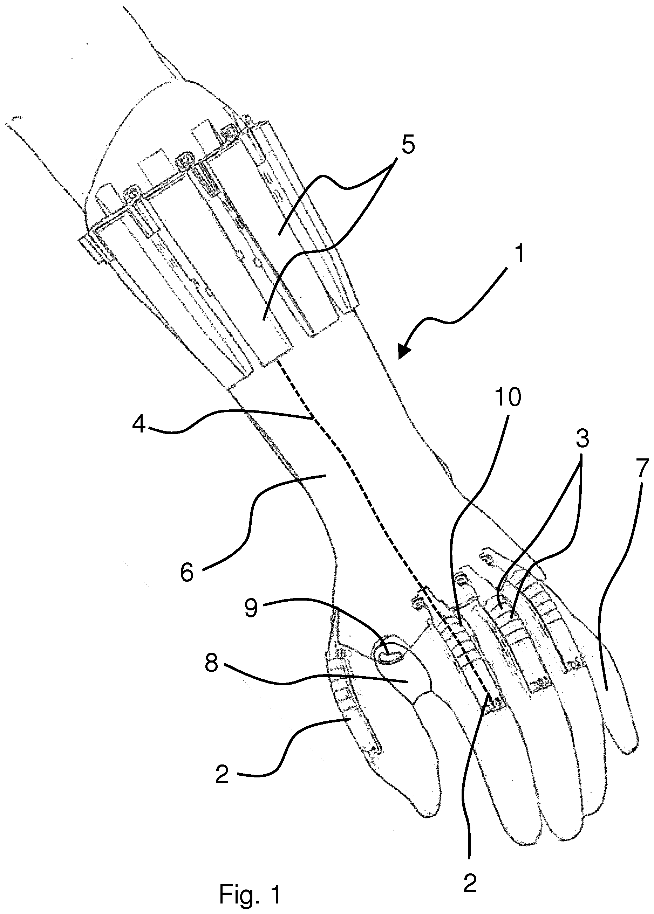

Figur 1 zeigt in einer perspektivischen Ansicht eine Handorthese 1 zum Beugen und/oder Strecken mindestens eines Fingers einer Patientenhand. Die Handorthese 1 umfasst dabei mehrere Fingersegmente 2, die jeweils aus einer Mehrzahl von gelenkig miteinander verbundenen Modulgliedern 3 gebildet sind. Die Fingersegmente 2 sind dabei jeweils an ihren distalen Enden über als Stäbe geformte Kopplungselemente 4 mit einem als Servomotor gebildeten Krafteinleitungsmittel 5 verbunden, das an einer am Unterarm eines Patienten befestigbaren Schiene 6 befestigt ist. Die Kopplungselemente 4, von denen in der Figur 1 lediglich eines gestrichelt dargestellt ist, sind dabei in der Schiene 6 und den Fingersegmenten 2 verdeckt geführt. Dem distalen Ende der Schiene 6 ist ein Handschuhabschnitt 7 zugeordnet, auf dem die Fingersegmente 2 befestigt sind. Wird nun von dem Krafteinleitungsmittel 5 über die Kopplungselemente 4 eine Zug- oder eine Druckkraft auf das Fingersegment ausgeübt, so sorgt dies im Falle einer Zugkraft für ein Strecken und im Falle eine Druckkraft für ein Beugen der Fingersegmente und damit letztlich auch der damit verbundenen Finger. Zur Stützung der Handinnenfläche ist eine Palmarstütze 8 vorgesehen, die den Handschuhabschnitt 7 auf dessen der Handfläche zugewandten Seite umgreift und die an dem distalen Ende der Schiene 6 durch eine Rastverbindung 9 lösbar befestigt ist. Die einzelnen Modulglieder 3 sind bei der in der Figur 1 dargestellten Handorthese 1 durch eine Bajonettverbindung 10 gelenkig miteinander verbunden, die neben einem Abwinkeln auch eine translatorische Verstellung der über die Bajonettverbindung 10 verbundenen Modulglieder 3 relativ zueinander ermöglicht.

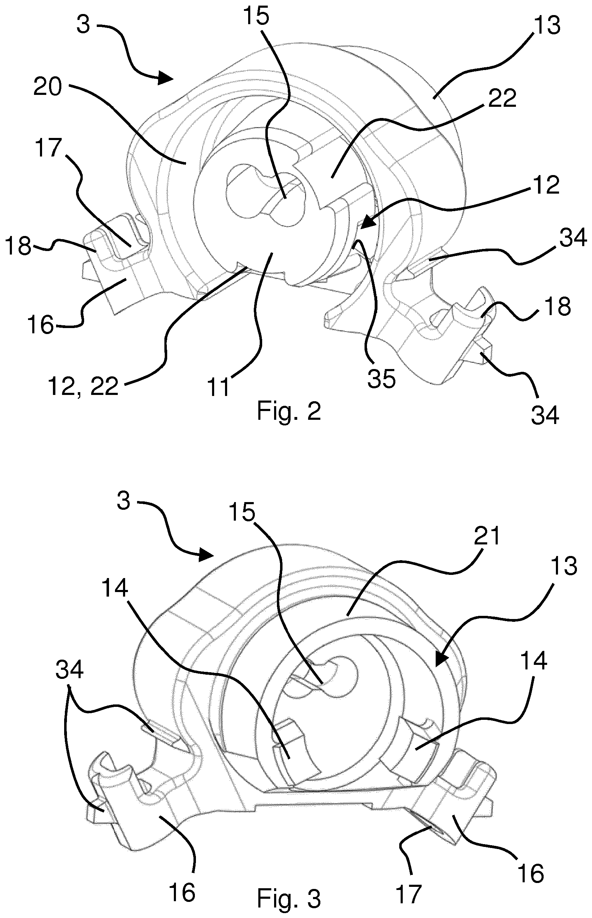

Wie den Figuren 2 und 3 entnommen werden kann, sind für die Bajonettverbindung 10 an einer ersten Stirnseite 11 der Modulglieder 3 Bajonettkulissen 12 ausgebildet, während an einer zweiten Stirnseite 13, die in der Figur 3 zu erkennen ist, korrespondierende Bajonetthaken 14 ausgebildet sind. Die Bajonetthaken 14 und die Bajonettkulissen 12 sind dabei auf einem gedachten Kreis nicht gegenüberliegend angeordnet, sondern weisen einen Winkel zueinander auf, der in dem gezeigten Ausführungsbeispiel rund 120° beträgt. Hierdurch wird durch die Bajonettverbindung 10 auch gleichzeitig ein Gelenk gebildet. Die Modulglieder weisen Durchführungen 15 für die Kopplungselemente 4 auf, die die von den Krafteinleitungsmitteln 5 erzeugten Stellkräfte auf die Fingersegmente 2 übertragen, um diese zu beugen und/oder zu strecken. Die Durchführungen 15 sind dabei gegenüber der Bajonettverbindung 10, insbesondere gegenüber den Bajonetthaken 14 nach oben versetzt, um eine bessere Kraftübertragung zu gewährleisten, sind also möglichst weit vom Drehzentrum entfernt. Als wirksame Hebelarme überlagern sich dabei der Abstand der Kopplungselemente 4 zu den Bajonetthaken 14 sowie der Abstand der Fingersegmente 2 zum Drehpunkt des Fingergelenks und der Abstand der Kopplungselemente 4 zu den Flügeln. Seitlich an den Modulgliedern 3 sind zudem Flügel 16 zu erkennen, die zur Verankerung der Modulglieder 3 in dem Handschuhabschnitt 7 dienen. Die Flügel 16 weisen dabei jeweils eine Öffnung 17 auf und sind zur Bildung eines Widerhakens 18 hakenförmig gebildet, wodurch die die Modulglieder 3 besser in dem Handschuhabschnitt 7 verankert werden können. Um bei einem Abwinkeln oder einer translatorischen Bewegung benachbarter Modulglieder 3 relativ zueinander eine Überdachung 19 zu erzielen, ist an der ersten Stirnseite 11 eine Aufnahme 20 ausgebildet, in die ein an der zweiten Stirnseite 13 benachbarter Modulglieder 3 ausgebildeter Vorsprung 21 eingreifen kann. Werden nun die über die Bajonettverbindung 10 verbundenen benachbarten Modulglieder 3 gegeneinander verkippt, so führt die Überdachung 19 dazu, dass hierbei kein Spalt entsteht und sorgt gleichzeitig dafür, dass die Bajonettverbindung 10 sich nicht unbeabsichtigt löst. Um überschüssiges Silikonmaterial beim Einbetten der Modulglieder 3 in den Handschuhabschnitt 7 abstreifen und entfernen zu können, sind Abstreifhilfen 34 vorgesehen. Beim Einbetten kann somit überschüssiges Silikonmaterial einfach - beispielsweise mittels eines auf den Abstreifhilfen aufgelegten Spatels abgestreift werden.

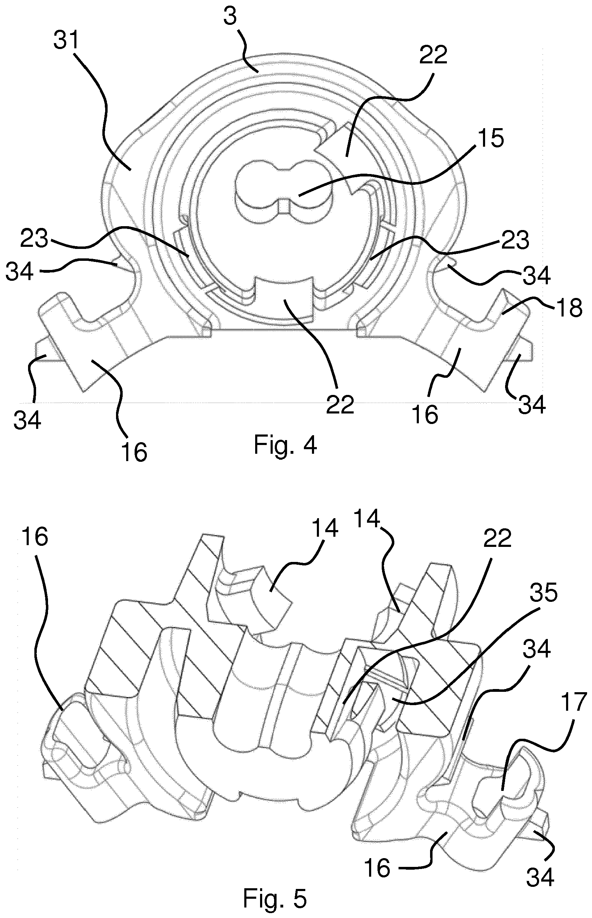

Aus den in den Figuren 4 und 5 dargestellten Ansichten des Modulgliedes 3 wird insbesondere der Verlauf der Bajonettkulisse 12 ersichtlich, der in dem gezeigten Ausführungsbeispiel eine Relativdrehung benachbarter Modulglieder 3 um einen Winkel von 65° erforderlich macht, um die Bajonettverbindung 10 zu schließen oder wieder zu öffnen. Mit anderen Worten sind die Einführöffnungen 22 der Bajonettkulisse 12 gegenüber der finalen Position 23 um einen Winkel von rund 65° verdreht, so dass auch zum Lösen der Bajonettverbindung 10 benachbarte Modulglieder 3 um diesen Winkel verstellt werden müssen, wodurch die Gefahr einer unbeabsichtigten Lösung der Bajonettverbindung 10 reduziert wird. In der finalen Position 23 befinden sich die Bajonetthaken 14 in Hakenaufnahmen 35 der Bajonettkulissen 12, in denen sich die Bajonetthaken 14 begrenzt bewegen können, so dass eine Verstellung benachbarter Modulglieder 3 in axialer Richtung ermöglicht ist. Die Hakenaufnahme 35 ist auch aus der Figur 2 ersichtlich. Der Figur 4 lässt sich auch entnehmen, dass das Modulglied 3 eine Verstärkung 31 aufweist, die das Modulglied 3 seitlich im Vergleich zu dessen Oberseite lokal verstärkt.

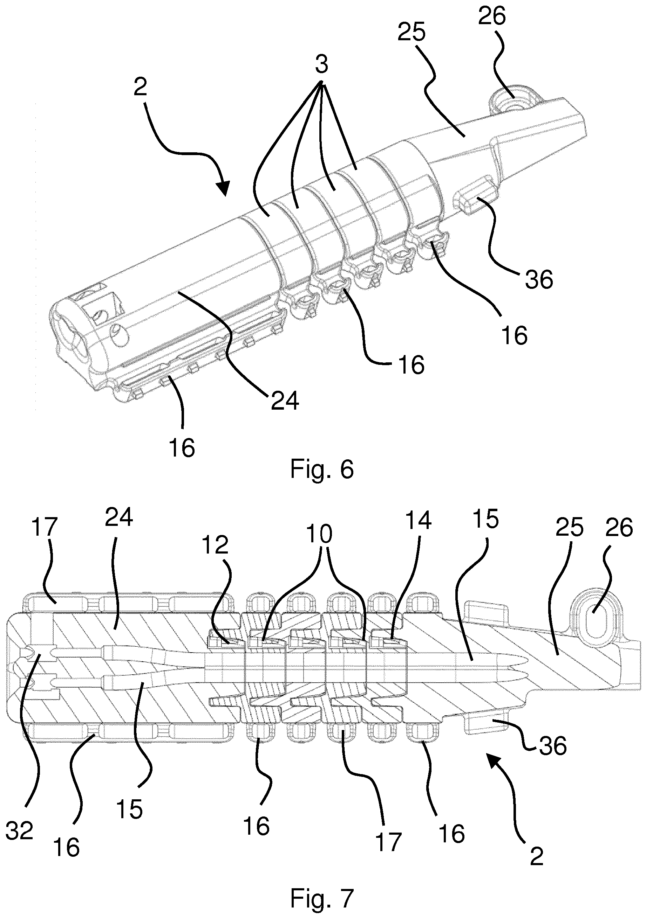

Figur 6 zeigt in einer perspektivischen Ansicht ein Fingersegment 2 der Handorthese 1. Hieraus ist ersichtlich, dass das Fingersegment 2 neben den Modulgliedern 3 auch ein distales Endstück 24 und ein proximales Endstück 25 umfasst, wobei das proximale Endstück 25 an der Schiene 6 und das distale Endstück 24 an dem Handschuhabschnitt 7 befestigt werden. Hierzu sind an dem distalen Endstück 24 ebenfalls Flügel 16 ausgebildet, mit denen das distale Endstück 24 in den Handschuhabschnitt 7 eingebettet werden kann. Das proximale Endstück 25 weist hingegen zusätzlich - neben den Flügeln 16 - auch eine Schraubenaufnahme 26 auf, mit der das Fingersegment 2 an der Schiene 6 festgeschraubt werden kann. Zudem sind dem proximalen Endstück 25 auch Einführhilfen 36 zugeordnet, die in korrespondierende Montageaufnahmen 37 eingeführt werden können, die in der Schiene 6 ausgebildet sind, wodurch die Fingersegmente 2 an der Schiene 6 befestigt werden.

Wie der in der Figur 7 dargestellten Schnittansicht durch das Fingersegment 2 entnommen werden kann, sind in den Modulgliedern 3 aber auch in dem distalen Endstück 24 und in dem proximalen Endstück 25 Durchführungen 15 ausgebildet, in denen die Kopplungselement 4, die mit dem Krafteinleitungsmittel 5 verbunden sind, zu dem distalen Endstück 24 geführt werden. Am distalen Endstück 24 werden die Kopplungselemente 4 dann über eine Schraubverbindung 32 gesichert. Der Figur 7 ist weiterhin auch zu entnehmen, dass an dem proximalen Ende des distalen Endstücks 24 eine Bajonettkulisse 12 ausgebildet ist, während an dem distalen Ende des proximalen Endstücks 25 Bajonetthaken 14 vorgesehen sind, um die Modulglieder 3 zwischen den beiden Endstücken 24, 25 zu befestigen.

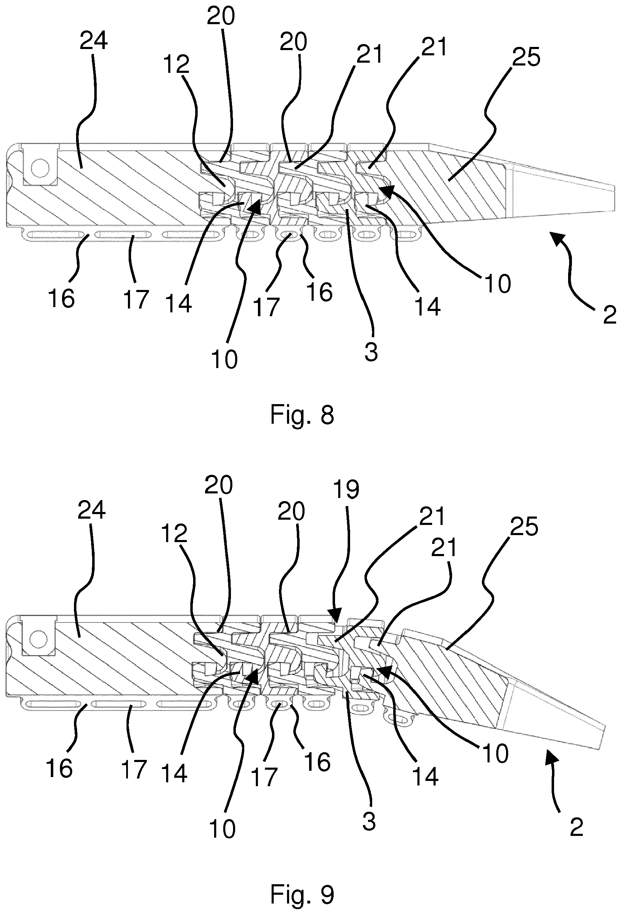

Die in den Figuren 8 und 9 dargestellten Längsschnitte durch das Fingersegment 2 zeigen, dass durch die Bajonettverbindung 10 eine translatorische Verstellung benachbarter Modulglieder 3 möglich ist. Dies ist insbesondere beim Beugen eines Fingers notwendig, um die Längenänderung zu kompensieren, die durch die beim Beugen auftretende Dehnung der Haut auf der Oberseite des Fingers bedingt ist. Auch wird insbesondere aus der in der Figur 9 dargestellten gebeugten Ansicht des Fingersegments 2 deutlich, dass durch die Wechselwirkung von Aufnahme 20 und Vorsprung 21 die Überdachung 19 gebildet wird, die verhindert, dass sich die Kopplungselemente 4 bei zu hohem Druck auswölben und dass Fremdkörper in das Innere der Mechanik gelangen. Als Drehpunkt beim Abwinkeln der Fingersegmente 2 wirkt dabei zunächst der gedachte Drehpunkt in der Silikoneinbettung. Sobald das Abwinkeln und die translatorische Verschiebung dazu führen, dass der Bajonetthaken 14 gegen den Anschlag des benachbarten Modulgliedes 3 läuft, wirkt er als zusätzlicher, überlagerter Drehpunkt und gleichzeitig als Anschlagsbegrenzung sowohl für die translatorische Verschiebung als auch für das Abwinkeln. Hierdurch wird verhindert, dass sich die Bajonettverbindung 10 zu weit öffnet, was ein Herausspringen benachbarter Modulglieder 3 zufolge haben könnte.

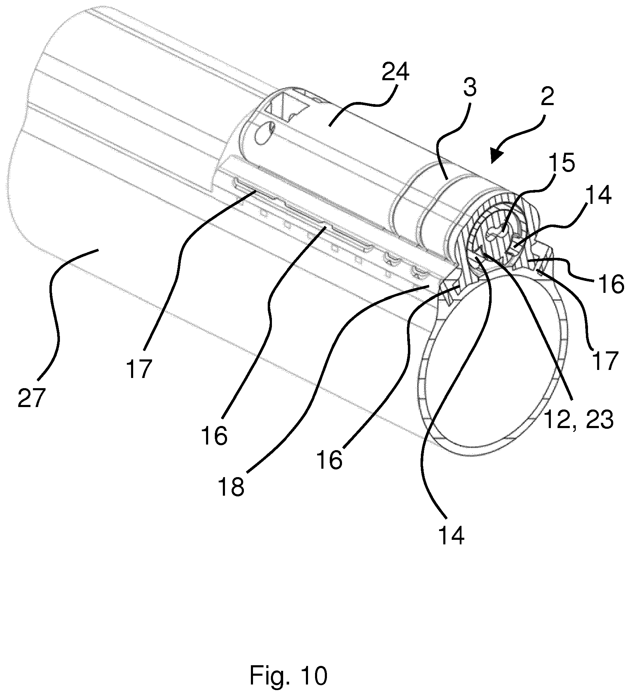

Figur 10 zeigt eine perspektivische Ansicht auf einen Fingerling 27 des Handschuhabschnitts 7, an dem ein Fingersegment 2 befestigt ist. Sowohl das in der Zeichnung dargestellte distale Endstück 24 als auch die Modulglieder 3 sind dabei in das Material des Fingerlings 27 eingebettet, das hierzu insbesondere die Flügel 16 mit den Öffnungen 17 und den Widerhaken 18 umgibt.

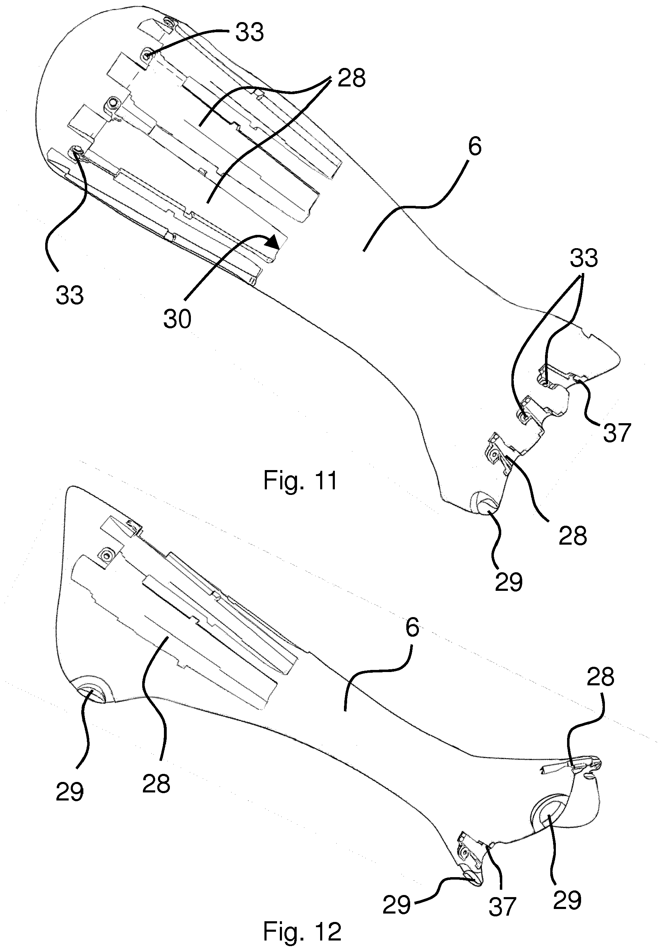

In der Figur 11 ist in einer Draufsicht und in der Figur 12 in einer Seitenansicht die Schiene 6 dargestellt. Hieraus wird insbesondere deutlich, dass in der Schiene 6 Aussparungen 28 eingearbeitet sind zur Aufnahme der Krafteinleitungsmittel 5 und der proximalen Endstücke 25 der Fingersegmente 2. Um die Krafteinleitungsmittel 5 und die proximalen Endstücke 25 in den Aussparungen zu befestigen, sind in die Schiene 6 Gewindebuchsen 33 eingearbeitet, in die die Krafteinleitungsmittel 5 und die proximalen Endstücke 25 verschraubt werden können. Auch sind Rastsitze 29 der Rastverbindung 9 zu erkennen, in denen die Palmarstütze 8 befestigt wird. Zudem ist insbesondere der Figur 12 zu entnehmen, dass im proximalen Bereich der Schiene 6 ein weiterer Rastsitz 29 ausgebildet ist, in dem ein Befestigungsmittel - beispielsweise ein Band - eingerastet werden kann, um die Schiene 6 am Arm des Patienten zu befestigen. In der Schiene 6 verlaufen zudem verdeckte Kanäle 30, in denen die Kupplungselemente 4 aufgenommen sind, die die Zug- und Druckkräfte von dem Krafteinleitungsmittel 5 auf die Fingersegmente 2 übertragen.

Im Folgenden wird das Herstellungsverfahren der Handorthese 1 noch einmal beschrieben. Zunächst wird der Handschuhabschnitt 7 individuell gefertigt und zwar basierend auf einem Gipsmodell der Patientenhand, auf das Silikon aufgetragen wird. Hierbei lassen sich in die Oberfläche des Handschuhabschnitts 7 auch beispielsweise Gewindebuchsen 33 einarbeiten die der Befestigung des Handschuhabschnitts 7 an der Schiene 6 dienen. Durch die Auswahl der Anzahl der verwendeten Modulglieder 3 werden die einzelnen Fingersegmente 2 an die Länge der Finger der Patientenhand angepasst. Dann werden die Fingersegmente 2 - also die Modulglieder 3 und die distalen Endstücke 24 sowie die proximalen Endstücke 25 - in das Obermaterial des Handschuhabschnitts 7 eingebettet, das vorzugsweise aus Silikon gefertigt ist. Dies erfolgt vorteilhafterweise dadurch, dass die Fingersegmente 2 mit den Flügeln 16 im Silikonmaterial verankert werden, diese also vom Silikon umgeben sind, wobei dabei das Silikonmaterial die Öffnungen 17 der Flügel 16 durchdringt. Die Widerhaken 18 bieten dabei zusätzliche Stabilität. Hierzu werden auf die Unterseite der Flügel 16 zunächst Silikonstreifen aufgetragen und durch die Zwischenräume und Öffnungen 17 gedrückt. Die Fingersegmente 2 werden dann auf den Silikonhandschuh aufgedrückt und anschließend von der Oberseite her ebenfalls mit Silikon gefüllt. Überschüssiges Silikonmaterial, das über die Abstreifhilfen 34 übersteht, wird dabei mittels Spatel von den Abstreifhilfen 34 abgestreift. Dann wird mittels eines 3D Scanners die Physiologie des Unterarms und vorteilhafterweise zumindest eines Teils der Handoberseite des Patienten erfasst, wobei der Patient hier bereits den zuvor gefertigten Handschuhabschnitt 7 trägt. Auf Grundlage dieser Daten wird eine Schiene 6, die das Handgelenk mit abdecken kann, durch ein generatives Herstellungsverfahren - vorzugsweise durch einen 3D Drucker - hergestellt. Bei dieser Herstellung werden bereits die für Befestigung der Fingersegmente 2 sowie der Krafteinleitungsmittel 5 benötigten Aussparungen 28 und die Kanäle 30 eingearbeitet Ebenfalls werden bereits zu diesem Zeitpunkt die Rastsitze 29, die für die Befestigung der Palmarstütze 8 vorgesehen sind, integral gefertigt. Die Gewindebuchsen 33 werden dann nachträglich in die Schiene 6 eingearbeitet. Die proximalen Endstücke 25 der Fingersegmente 2 werden an dem distalen Ende der Schiene 6 in den hierfür vorgesehenen Aussparung 28 befestigt. Hierzu werden die an den proximalen Endstücken 25 ausgebildeten Einführhilfen 36 in die Montageaufnahmen 37 der Schiene 6 eigeführt und mit der Schraubenaufnahme 26 an der Schiene 6 festgeschraubt. Dann wird in einem letzten Schritt die Palmarstütze 8 an die individuelle Handinnenfläche des Patienten angepasst. Hierzu hat es sich bewährt, ein Niedertemperaturthermoplast-Material zu verwenden, da dieses einfach modelliert und an die Physiologie der Patientenhand angepasst werden kann. Hierbei werden dann auch die für die Rastverbindung 9 mit der Schiene 6 benötigten Strukturen ausgebildet.

### Bezugszeichenliste

- 1: Handorthese
- 2: Fingersegment
- 3: Modulglied
- 4: Kopplungselement
- 5: Krafteinleitungsmittel
- 6: Schiene
- 7: Handschuhabschnitt
- 8: Palmarstütze
- 9: Rastverbindung
- 10: Bajonettverbindung
- 11: erste Stirnseite
- 12: Bajonettkulisse
- 13: zweite Stirnseite
- 14: Bajonetthaken
- 15: Durchführung
- 16: Flügel
- 17: Öffnung
- 18: Widerhaken
- 19: Überdachung
- 20: Aufnahme
- 21: Vorsprung
- 22: Einführöffnung
- 23: finale Position
- 24: distales Endstück
- 25: proximales Endstück
- 26: Schraubenaufnahme
- 27: Fingerling
- 28: Aussparung
- 29: Rastsitz
- 30: Kanal
- 31: Verstärkung
- 32: Schraubverbindung
- 33: Gewindebuchse
- 34: Abstreifhilfe
- 35: Hakenaufnahme
- 36: Einführhilfe
- 37: Montageaufnahme

## Patentansprüche

1. Modulglied (3) zur Verwendung in einer Handorthese (1) mit mindestens einer an einer ersten Stirnseite (11) ausgebildeten Bajonettkulisse (12), **gekennzeichnet durch** mindestens einen zu der Bajonettkulisse (12) korrespondierenden Bajonetthaken (14), der an einer zweiten Stirnseite (13) ausgebildet ist, zur Bildung einer Bajonettverbindung (10) zwischen benachbarten Modulgliedern (3).

2. Modulglied (3) nach Anspruch 1, **dadurch gekennzeichnet, dass** die Bajonettkulisse (12) eine Hakenaufnahme (35) umfasst, die derart ausgebildet ist, dass ein sich in einer finalen Position (23) befindender, benachbarter Bajonetthaken (14) in axialer Richtung des Modulglieds (3) begrenzt bewegen kann.

3. Modulglied (3) nach Anspruch 1 oder 2, **dadurch gekennzeichnet, dass** die Bajonettverbindung (10) zwischen benachbarten Modulgliedern (3) ein Bajonettgelenk realisiert, das ausgebildet ist, eine Verkippung benachbarter Modulglieder (3) zu ermöglichen, wozu die Bajonetthaken (14) und die Bajonettkulissen (12) einen, das Verkippen benachbarter Modulglieder (3) begrenzenden, Anschlag und korrespondierenden Gegenanschlag bilden.

4. Modulglied (3) nach einem der Ansprüche 1 bis 3, **dadurch gekennzeichnet, dass** die Bajonettkulisse (12) und der Bajonetthaken (14) jeweils mehrfach vorgesehen und unter einem Winkel zueinander angeordnet sind, der zwischen 40° und 170° liegt.

5. Modulglied (3) nach Anspruch 4, **dadurch gekennzeichnet, dass** der Winkel zwischen 60° und 160° liegt.

6. Modulglied (3) nach Anspruch 4 oder 5, **dadurch gekennzeichnet, dass** der Winkel bei 120° liegt.

7. Modulglied (3) nach einem der Ansprüche 1 bis 6, **dadurch gekennzeichnet, dass** an der ersten Stirnseite (11) eine Aufnahme (20) ausgebildet ist, dass an der zweiten Stirnseite (13) ein Vorsprung (21) ausgebildet ist, derart, dass durch die Aufnahme (20) und den Vorsprung (21) zwischen benachbarten Modulgliedern (3) eine Überdachung (19) gebildet wird.

8. Modulglied (3) nach einem der Ansprüche 1 bis 7, **dadurch gekennzeichnet, dass** dem Modulglieder (3) mindestens ein Flügel (16) zugeordnet ist zur Verankerung des Modulglieds (3) in einem Handschuhabschnitt (7) einer Handorthese (1).

9. Modulglied (3) nach Anspruch 8, **dadurch gekennzeichnet, dass** dem Flügel (16) jeweils eine Öffnung (17) zugeordnet ist.

10. Modulglied (3) nach Anspruch 8 oder 9, **dadurch gekennzeichnet, dass** dem Flügel (16) jeweils ein Widerhaken (18) zugeordnet ist.

11. Modulglied (3) nach einem der Ansprüche 1 bis 10, **dadurch gekennzeichnet, dass** Abstreifhilfen (34) vorhanden sind, um überschüssiges Silikonmaterial beim Einbetten des Modulglieds (3) abzustreifen und zu entfernen.

12. Modulglied (3) nach einem der Ansprüche 1 bis 11, **dadurch gekennzeichnet, dass** eine Verstärkung (31) vorhanden ist, die das Modulglied (3) seitlich im Vergleich zu dessen Oberseite lokal verstärkt.

13. Modulglied (3) nach einem der Ansprüche 1 bis 10, **dadurch gekennzeichnet, dass** Durchführungen (15) für Kopplungselemente (4) einer Handorthese (1) vorhanden sind.

14. Kombination aus einem ersten Modulglied (3) nach einem der Ansprüche 1 bis 13 und einem zweiten Modulglied (3) nach einem der Ansprüche 1 bis 13.

## Claims

1. Module element (3) for use in a hand orthosis (1) with at least one bayonet link (12) formed on a first end face (11), **characterized by** at least one bayonet hook (14) corresponding to the bayonet link (12), which is formed on a second end face (13), for forming a bayonet connection (10) between adjacent module elements (3).

2. Module element (3) according to claim 1, **characterized in that** the bayonet link (12) comprises a hook receptacle (35), which is designed in such that an adjacent bayonet hook (14) located in a final position (23) can move in the axial direction of the module element (3) in a limited manner.

3. Module element (3) according to claim 1 or 2, **characterized in that** the bayonet connection (10) between adjacent module elements (3) implements a bayonet joint which is designed to allow tilting of adjacent module elements (3), for which purpose the bayonet hooks (14) and the bayonet linkages (12) form a stop limiting the tilting of adjacent module element (3) and a corresponding counterstop.

4. Module elements (3) according to one of claims 1 to 3, **characterized in that** the bayonet link (12) and the bayonet hook (14) are each provided several times and are arranged at an angle to one another which is between 40° and 170°.

5. Module element (3) according to claim 4, **characterized in that** the angle is between 60° and 160°.

6. Module element (3) according to claim 4 or 5, **characterized in that** the angle is at 120°.

7. Module element (3) according to one of claims 1 to 6, **characterized in that** a receptacle (20) is formed on the first end face (11), **in that** a projection (21) is formed on the second end face (13) in such a way that a canopy (19) is formed by the receptacle (20) and the projection (21) between adjacent module elements (3).

8. Module element (3) according to one of claims 1 to 7, **characterized in that** at least one wing (16) is assigned to the module element (3) for anchoring the module element (3) in a glove section (7) of a hand orthosis (1).

9. Module element (3) according to claim 8, **characterized in that** an opening (17) is assigned to each wing (16).

10. Module element (3) according to claim 8 or 9, **characterized in that** a barb (18) is assigned to each wing (16).

11. Module element (3) according to one of claims 1 to 10, **characterized in that** stripping aids (34) are present in order to strip off and remove excess silicone material when embedding the module element (3).

12. Module element (3) according to one of claims 1 to 11, **characterized in that** a reinforcement (31) is present, which locally reinforces the module element (3) laterally in comparison with its upper side.

13. Module element (3) according to one of claims 1 to 10, **characterized in that** lead-throughs (15) for coupling elements (4) of a hand orthosis (1) are present.

14. Combination of a first module element (3) according to one of claims 1 to 13 and a second module element (3) according to one of claims 1 to 13.

## Revendications

1. Membre modulaire (3) à utiliser dans une orthèse de main (1) avec au moins une coulisse à baïonnette (12) réalisée sur une première face frontale (11), **caractérisé par** au moins un crochet à baïonnette (14) correspondant à la coulisse à baïonnette (12), qui est réalisé sur une seconde face frontale (13) pour former une liaison à baïonnette (10) entre des membres modulaires (3) voisins.

2. Membre modulaire (3) selon la revendication 1, **caractérisé en ce que** la coulisse à baïonnette (12) comprend un logement de crochet (35) qui est réalisé de sorte qu'un crochet à baïonnette (14) voisin se trouvant dans une position finale (23) puisse se déplacer de manière limitée dans la direction axiale du membre modulaire (3).

3. Membre modulaire (3) selon la revendication 1 ou 2, **caractérisé en ce que** la liaison à baïonnette (10) entre des membres modulaires (3) voisins crée une articulation à baïonnette qui est réalisée pour permettre un basculement de membres modulaires (3) voisins, pour lequel les crochets à baïonnette (14) et les coulisses à baïonnette (12) forment une butée limitant le basculement de membres modulaires (3) voisins et une contrebutée correspondante.

4. Membre modulaire (3) selon l'une quelconque des revendications 1 à 3, **caractérisé en ce que** la coulisse à baïonnette (12) et le crochet à baïonnette (14) sont respectivement prévus en plusieurs exemplaires et sont disposés selon un angle l'un par rapport à l'autre qui est compris entre 40° et 170°.

5. Membre modulaire (3) selon la revendication 4, **caractérisé en ce que** l'angle est compris entre 60° et 160°.

6. Membre modulaire (3) selon la revendication 4 ou 5, **caractérisé en ce que** l'angle est de 120°.

7. Membre modulaire (3) selon l'une quelconque des revendications 1 à 6, **caractérisé en ce qu'**un logement (20) est réalisé sur la première face frontale (11), **en ce qu'**une saillie (21) est réalisée sur la deuxième face frontale (13), de telle sorte qu'un toit (19) est formé par le logement (20) et la saillie (21) entre des membres modulaires (3) voisins.

8. Membre modulaire (3) selon l'une quelconque des revendications 1 à 7, **caractérisé en ce qu'**au moins une aile (16) est associée au membre modulaire (3) pour l'ancrage du membre modulaire (3) dans une section de gant (7) d'une orthèse de main (1).

9. Membre modulaire (3) selon la revendication 8, **caractérisé en ce qu'**une ouverture (17) est associée respectivement à une aile (16).

10. Membre modulaire (3) selon la revendication 8 ou 9, **caractérisé en ce qu'**un crochet (18) est associé respectivement à une aile (16).

11. Membre modulaire (3) selon l'une quelconque des revendications 1 à 10, **caractérisé en ce que** des moyens de raclage (34) sont prévus pour racler et éliminer le matériau silicone en excès lors de l'enrobage du membre modulaire (3).

12. Membre modulaire (3) selon l'une quelconque des revendications 1 à 11, **caractérisé en ce qu'**un renfort (31) est prévu pour renforcer localement le membre modulaire (3) latéralement par rapport à sa face supérieure.

13. Membre modulaire (3) selon l'une quelconque des revendications 1 à 10, **caractérisé en ce que** des passages (15) sont prévus pour des éléments de couplage (4) d'une orthèse de main (1).

14. Combinaison d'un premier membre modulaire (3) selon l'une quelconque des revendications 1 à 13 et d'un second membre modulaire (3) selon l'une quelconque des revendications 1 à 13.
